# EUROPÄISCHE PATENTSCHRIFT

(11) **EP 2 206 706 B1**
(45) Veröffentlichungstag und Bekanntmachung des Hinweises auf die Patenterteilung: **10.07.2013**
(21) Anmeldenummer: 09178920.6
(22) Anmeldetag: 11.12.2009
(51) Int. Cl.: C07D 265/36

(54) **Verfahren zur Herstellung von Acylamid-Verbindungen**
Method for producing acylamide compounds
Procédé destiné à la production de composés d'acylamide

(30) Priorität: 23.12.2008 DE 102008062905
(43) Veröffentlichungstag der Anmeldung: 14.07.2010
(73) Patentinhaber: Saltigo GmbH, 40764 Langenfeld (DE)
(72) Erfinder: Boll, Matthias, 51061 Köln (DE); Koch, Burkhard, 50823 Köln (DE)
(74) Vertreter: Pettrich, Klaus-Günter

(56) Entgegenhaltungen:
- WO-A1-01/90088
- HAYAKAWA I ET AL: "SYNTHESIS AND ANTIBACTERIAL ACTIVITIES OF SUBSTITUTED 7-OXO-2,3-DIHYDRO-7H-PYRIDOÚ1,2,3-DE 3/4 Ú1,4 3/4 BENZOXAZINE-6-CARBOXYLIC ACIDS", CHEMICAL AND PHARMACEUTICAL BULLETIN, PHARMACEUTICAL SOCIETY OF JAPAN, TOKYO, JP, Bd. 32, Nr. 12, 1. Dezember 1984 (1984-12-01), Seiten 4907-4913, XP000654032, ISSN: 0009-2363
- SMITH A J ET AL: "THE PREPARATION OF SKELETAL CATALYSTS", ANNUAL REVIEW OF MATERIALS RESEARCH, ANNUAL REVIEWS, PALO ALTO, CA, US, Bd. 35, 1. August 2005 (2005-08-01), Seiten 127-135, XP009066046, ISSN: 1531-7331
- MU XUHONG ET AL: "Amorphous Nickel based alloy catalysts and magnetically stabilized bed hydrogenation technology", CHINA PETROLEUM PROCESSING AND PETROCHEMICAL TECHNOLOGY, CN, Bd. 4, 1. Dezember 2002 (2002-12-01), Seiten 25-31, XP009130837, ISSN: 1008-6234

## Beschreibung

Beschrieben wird ein Verfahren zur Herstellung von Acylamid-Verbindung, bei dem eine gereinigte o-Nitrophenoxycarbonylverbindung mit Wasserstoff-Gas in Gegenwart eines Sponge-metal-Katalysators unter Ringschluss zu einem Benzoxazin hydriert wird, welches anschließend mit einem Acylhalogenid zur entsprechenden Acylamid-Verbindung umgesetzt wird.

Acylamide sind in der Farbstoffindustrie und in agrochemischen Produkten wie z.B. 4-(Dichloracetyl)-3,4-dihydro-3-methyl-2H-1,4-benzoxazin (Benoxacor) zu finden. WO 01/90088 offenbart die Herstellung von Acylamid- Verbindungen ohne Kontrolle des pH-Wertes.

Die Herstellung von Benoxacor, einem Safener beim Einsatz von Herbiziden, ist ein dreistufiger Prozess: In der ersten Stufe wird o-Nitrophenol mit Chloraceton zu o-Nitrophenoxyaceton umgesetzt, in der zweiten Stufe wird die Verbindung hydriert, so dass ein Benzoxazin erhalten wird, das anschließend noch acyliert wird. Dieses Verfahren ist in der US2006/0017946A1 beschrieben.

Der in diesem Verfahren geschwindigkeitsbestimmende und damit teuerste Schritt ist die Hydrierung der o-Nitrophenoxycarbonylverbindung, hier ein Nitrophenoxyketon, zum korrespondierenden Benzoxazin. In dem in US2006/0017946A1 beschriebenen Verfahren wird hierzu ein Edelmetallkontakt eingesetzt, der auf einen Aktivkohlefilter aufgezogen wurde. Mit diesem Verfahren sind laut vorliegender Beschreibung mehrere Nachteile verbunden:
- Zum einen handelt es sich um einen Batch-Prozess, der mit einer geringen Raum-Zeit-Ausbeute einhergeht und daher kostenintensiv ist,
- des weiteren wird ein verhältnismäßig teurer Katalysator eingesetzt, der dazu noch eine hohe Beladung eines sehr wertvollen Edelmetalls aufweist (von etwa 5 Gew.-% Pt),
- des weiteren wird der eingesetzte Katalysator nach nur etwa 10g Nitroverbindung pro Gramm Katalysator in einem aufwändigen Waschschritt von Nebenkomponenten gereinigt, was neben einer weiteren Verlängerung der Standzeit auch Kosten für das Recycling bzw. die Entsorgung der verwendeten Lösungsmittel verursacht,
- des weiteren entsteht in dem dort vorgeschlagenen Verfahren eine Reihe von Nebenkomponenten, im besonderen das 3MBM-Dimer, das die Reinheit des Endproduktes und die Ausbeute beeinflusst.

Die im zweiten Schritt geforderte Reduktion von aromatischen Nitrogruppen mit Wasserstoff zu den entsprechenden Aminen als eine nicht isolierte Zwischenstufe wird in der chemischen Industrie weltweit großtechnisch durchgeführt. Grundsätzlich ist für Nitroreduktionen eine ganze Reihe von Katalysatoren geeignet, angefangen von Nickel- oder Kobaltkatalysatoren (in Form der entsprechenden Sponge-metal-Katalysatoren,(auch Skelett-Katalysatoren genannt) hergestellt aus Aluminium-Nickel-Legierungen durch Laugung oder auch feinverteilt auf Trägermaterialien). Gemische aus Nickel und anderen Metallen, wie zum Beispiel Eisen, Molybdän, Chrom (alle ebenfalls hergestellt durch Laugung von entsprechenden Aluminium-Metall-Legierungen) sind ebenfalls prinzipiell geeignet.

Diese Legierungen können auch bei niedriger Temperatur in wässriger Phase unter Zugabe von Ammoniumchlorid zur Nitroreduktion eingesetzt werden, wie Bhaumik (Canadian Journal of Chemistry (2003), 81(3), 197-198) berichtet.

Neben diesen vergleichsweise kostengünstigen Katalysatortypen kommen auch geträgerte Edelmetallkatalysatoren in Frage, zum Beispiel Platin oder Palladium auf Aktivkohle, die wie die Raney-Katalysatoren auch mit der Zugabe von Ammoniak oder einem anderen Amin oder einem andere Katalysatorgift gezielt in der Reaktivität gebremst werden können. Diese Katalysatoren haben dabei zwei Nachteile gegenüber den Nickel-Sponge-metal oder Kobalt-Sponge-metal Katalysatoren: Zum einen sind sie in der Regel schlechter filtrierbar und die damit verbundenen Katalysatorverluste sind größer, zum anderen sind die Anschaffungskosten für edelmetallhaltige Katalysatoren deutlich höher.

Andere Möglichkeiten, Nitrogruppen zu reduzieren, wie etwa die im Organikum (21. Auflage, S. 627 ff., Wiley VCH, Weinheim) vorgeschlagene Reduktion mit Zink, Zinn oder Eisen in HCl, spielen in der chemischen Industrie ebenso eine nur untergeordnete Rolle wie die Reduktion mit zum Beispiel Hydrazin, wie sie unter anderem von Han et al. (Tetrahedron Letters (1985), 26(50), 6233-4) beschrieben wurde. Auch homogene Katalysatoren auf Basis von Edelmetallen wurden für die Reduktion bereits erwähnt, zum Beispiel durch Sandra et al. (Journal of Molecular Catalysis (1987), 39(3), 279-92). In den oben angegebenen Beispiele wurde ausserdem immer nur die Nitrogruppe am Aromaten hydriert, eine weitere, reduktive Aminierung wurde hier nicht betrachtet.

Es gibt zurzeit auch die Möglichkeit, zur Reduktion Metallkatalysatoren auf einem anorganischen Träger zu verwenden, wie etwa einen Kupferkatalysator in Gegenwart von Nickel und / oder Palladium auf einem Silikat (CN1861253). Ebenso wurde durch Lu et al. (Zhejiang Gongye Daxue Xuebao (2002), 30(5), 464-466) von Carbonanotubes als Trägermaterial berichtet.

Limitiert wird der Einsatz von Sponge-metal-Katalysatoren vor allem durch die Temperatur, bei der die Hydrierung ablaufen kann: Bei zu hohen Temperaturen wird in der Regel nicht nur die Nitrogruppe, sondern auch unerwünschterweise der aromatische Kern hydriert. Diese Kernhydrierung führt zu Ausbeuteverlusten und, damit verbunden, zu höheren Kosten. Aufgabe der vorliegenden Erfindung war es, ein Verfahren zur Herstellung von Acylamid - Verbindungen aufzufinden, das sich in einer guten Raum-Zeit-Ausbeute mit nur einer geringen Bildung unerwünschter Nebenprodukte sowie mit kostengünstigen Katalysatoren durchführen lässt.

Es wurde nun ein Verfahren gefunden, das diese Anforderungen erfüllt.

Gegenstand der Erfindung ist daher ein Verfahren zur Herstellung einer Acylamid -Verbindung der allgemeinen Formel (I) 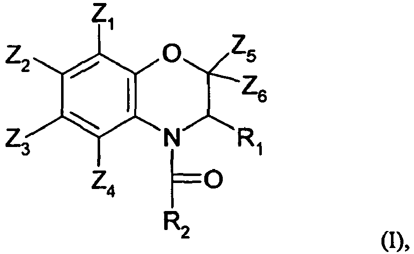 in der
- R₁: Wasserstoff oder ein C₁-C₈-Alkylrest, R₂ eine Dichlormethyl- oder Trichlorethylgruppe und Z₁ bis Z₆ jeweils unabhängig voneinander Wasserstoff oder ein C₁-C₈-Alkylrest sind,
dadurch gekennzeichnet, dass man
a) eine o-Nitrophenoxycarbonylverbindung der allgemeinen Formel (II) 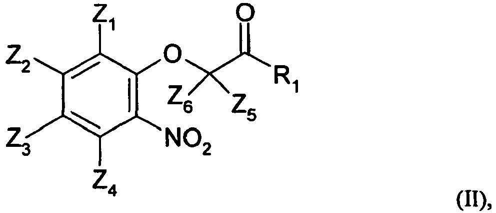 in der
   R₁ und Z₁ bis Z₆ die für Formel (I) angegebene Bedeutung haben,
   aus einem organischen Lösungsmittel auskristallisieren lässt und abtrennt,
b) die o-Nitrophenonoxycarboxylverbindung in einem Gemisch aus einem C₁-C₃-Alkohol und einem aromatischen Lösungsmittel löst,
c) den pH-Wert in dieser Lösung auf einen Wert im Bereich von 8 bis 9 einstellt und dann
d) die in dieser Lösung enthaltene o-Nitrophenoxyverbindung der Formel (II) in Gegenwart eines Sponge-metal-Katalysators mit Wasserstoff-Gas unter Ringschluss zur Benzoxazin-Verbindung der Formel (III) hydriert 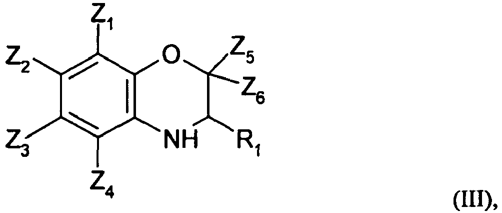 in der R₁ und Z₁ bis Z₆ die für Verbindungen der Formel(I) angegebene Bedeutung haben, und dann
e) die Verbindung der Formel (III) mit einem Acylhalogenid der Formel (IV) 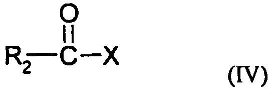 in der R₂ die für Verbindungen der Formel (I) angegebene Bedeutung hat und X für ein Halogenatom, insbesondere Chloratom steht, zu Verbindungen der Formel (I) umsetzt.

Das organische Lösungsmittel, in dem in Schritt a) die Kristallisation durchgeführt wird, kann ein Alkohol wie Methanol, Ethanol oder Isopropanol im Gemisch mit einem aromatischen Lösungsmittel wie Benzol, Toluol oder Xylol sein. Bevorzugt wird Isopropanol im Gemisch mit Toluol in Schritt a) verwendet. Der durch Kristallisation bis -5°C oder darunter erhaltene Feststoff wird dann in Schritt b) z.B. in einem Methanol/Toluol-Lösungsmittelgemisch gelöst und in Schritt c), z.B. durch Natriumhydroxid, auf einen pH zwischen 6 und 11 eingestellt, bevor in Schritt d) dann die Hydrierung bei einem Wasserstoffdruck von z.B. 2,2 · 10⁷ PA (220 bar) durchgeführt wird.

Für die Hydrierung der o-Nitrophenoxycarbonylverbindung konnte überraschenderweise gezeigt werden, dass sich diese Reaktion in Gegenwart von speziellen Sponge-metal-Katalysatoren praktisch ohne Nebenreaktionen (zum Beispiel zu Dimeren des Benzoxazin oder zu teilhydrierten Verbindungen oder zu anderen, unerwünschten Nebenprodukten) durchführen lässt, wenn man eine frisch aus- bzw. umkristallisierte o-Nitrophenoxycarbonylverbindung der Formel (II) in einem Gemisch aus einem C₁-C₃-Alkohol wie z.B. Methanol und einem aromatischen Lösungsmittel wie z.B. Toluol löst, bei dem der pH-Wert auf einen Wert zwischen 8-9 eingestellt wurde.

Der Einfluss des im Lösungsmittelgemisch gemessenen "pH-Wertes" (der Begriff pH-Wert muss im vorliegenden Fall eigentlich durch eine gemessene Spannung gegen eine Referenzelektrode ersetzt werden, da in einem nicht-wässrigen System gemessen wurde) hat einen überraschenden erheblichen Einfluss auf die Bildung von Nebenkomponenten und die Standzeit des Katalysators, wie Vergleichsversuche bei anderen pH-Werten zeigen. Üblicherweise wird der pH-Wert mit einer pH-Glaselektrode gemessen.

Im Allgemeinen führt man die Hydrierung in einem Lösungsmittelgemisch aus einem C₁-C₃-Alkohol wie Methanol, Ethanol oder Propanol im Gemisch mit einen aromatischen Lösungsmittel wie Toluol aus. Die Mischungsverhältnisse (in Gewichtsanteilen) liegen zwischen 1:100 aromatisches Lösungsmittel zu Alkohol bis zu 20:100 aromatisches Lösungsmittel zu Alkohol, so dass auch unter der Berücksichtigung des entstandenen Reaktionswasser bevorzugt (aber nicht notwendigerweise) eine Einphasigkeit des Reaktionsgemisches am Ende der Reaktion gewährleistet bleibt. Bevorzugt ist ein Toluol/Methanol -Gemisch als Lösungsmittel für die Hydrierung mit einem Mischungsverhältnis von 9,7:100 Toluol zu Methanol. In Anlehnung an die Beschreibung aus US2006/0017946A1 können aber auch Lösungsmittelgemische mit einem deutlich höheren Anteil an Toluol verwendet werden.

Außerdem zeigt der Katalysator unter den gewählten Versuchsbedingungen mit >90g Nitroverbindung pro Gramm Katalysator (ohne Zwischenwäsche des Katalysators) überraschend eine deutlich höhere Standzeit als ein edelmetallhaltiger Katalysator.

Als Sponge-Metal-Katalysatoren können solche eingesetzt werden, die überwiegend aus Nickel bestehen, es können aber auch mit Eisen, Aluminium oder Molybdän dotierte Sponge-Metal-Katalysatoren eingesetzt werden. Bevorzugt wird ein mit Molybdän dotierter Katalysator, z.B. mit 0.5 bis 4% Molybdän dotierter Nickel-Sponge-metal-Katalysator, wie er z.B. unter dem Handelsnamen AMPERKAT Ni-Mo 3706 von der Fa. H.C. Starck (Goslar, Deutschland) erhältlich ist, eingesetzt. Der Wasserstoffdruck liegt üblicherweise zwischen 1,010⁷ und 2,4 · 10⁷ Pa (100 und 240 bar), bevorzugt bei 2,2 · 10⁷ Pa (220 bar).

Die Reaktionstemperatur kann bei der Hydrierung in Schritt d) üblicherweise im Bereich zwischen 30 und 90 °C, bevorzugt bei 60 °C, liegen.

Die Reaktionsdauer für die Hydrierung sollte im Bereich zwischen 0,3 und 5 Stunden, bevorzugt bei 0,5 bis 1,5 Stunden, liegen.

Die Hydrierung kann diskontinuierlich oder bevorzugt kontinuierlich ausgeführt werden.

Die Acylierung in Schritt e) ist dem Fachmann im Prinzip bekannt. Üblicherweise wird die Lösung aus Schritt d) zur Abtrennung des Katalysators filtriert und mit Lösungsmittel, z.B. Toluol, verdünnt und dann mit einem Acylhalogen der Formel (IV) wie vorzugsweise Dichloressigsäurechlorid ( mit R ₂= Dichlormethyl in Formel (IV)) oder Trichlorpropionsäurechlorid (mit R₂ = Trichlorethyl in Formel (IV)) in toluolischer Lösung in Gegenwart von wässriger Natronlauge umgesetzt. Bevorzugt erfolgt die Umsetzung mit Dichloressigsäurechlorid (DCAC) bei einem pH-Wert von 2-3,5 bei Temperaturen bis zu 80°C.

Das erfindungsgemäße Verfahren eignet sich zur Herstellung aller bekannten Acylamide der Formel (I). Bevorzugt wird nach dem erfindungsgemäßen Verfahren aber 4-(Dichloracetyl)-3,4-dihydro-3-methyl-2H-1,4-benzoxazin (Benoxacor) aus o-Nitrophenoxyaceton hergestellt. Hierfür wird o-Nitrophenoxyaceton z.B. aus der Umsetzung von o-Nitrophenol mit Chloraceton (wie in Beispiel 1 angegeben) auskristallisiert und erneut gelöst und dann in Gegenwart von dotiertem Sponge-Metal-Katalysatoren mit Wasserstoff hydriert. Das so aus o-Nitrophenoxyaceton erhaltene Benzoxazin der Formel (III) mit R₁ = Methyl und Z₁ bis Z₆ = Wasserstoff ist 3,4-Dihydr-3-methyl-2H-1,4-benzoxazin (2-MBM wird mit DCAC dann zum 4-(Dichloracetyl)-3,4-dihydro-3-methyl-2H-1,4-benzoxazin (Benoxacor) umgesetzt, das aus einem Isopropanol-Toluol-Wasser-Gemisch auskristallisiert wird und als Safener Herbiziden beigegeben oder in Kobination mit solchen eingesetzt wird).

Die folgenden Beispiele sollen die Erfindung weiter erläutern, ohne sie jedoch in ihrem Umfang zu begrenzen.

### Beispiele

### 1. Stufe: Herstellung von o-Nitrophenoxyaceton (Vorstufe, Verbindung der Formel (II)

a) In einem mit Stickstoff gespülten 30 Liter Doppelmantelgefäß mit Rührer, zwei Pt100-Meßfühlern, pH-Elektrode, geerdetem HC4-Metallstab und Rückflusskühler wurden 3652g o-Nitrophenol 98,3%ig (25,81 mol) in 7910g Toluol gelöst. Zu dieser Lösung wurden 363g Natriumbromid (3,53 mol) 2630g (31,31 mol) Natriumhydrogencarbonat 217g 70%ige Tributylmethylammoniumchlorid-Lösung (0,64 mol) in Wasser zugegeben. Anschließend wurden in Stickstoffatmosphäre unter Rühren 2780g Chloraceton (30,05 mol, >96%ig) zugegeben. Die Mischung wurde unter Rühren in einer Stickstoffatmosphäre auf 65°C erhitzt, wobei sich viel CO₂ entwickelte.
b) Nachdem die Gasentwicklung fast zum Stillstand gekommen war (nach etwa sechs Stunden), wurden 5456g Wasser (55°C warm) zudosiert, dann mit 10%ige Salzsäure (~1396g) unter Rühren auf einen pH 6,9-7 eingestellt. Die wässrige Phase wurde abgetrennt und die organische Phase mit 5800g 10%ige Natriumchlorid-Lösung (55°C warm) gewaschen. Die wässrige Phase wurde wieder abgetrennt.
c) Die organische Phase wurde mit 4000g Isopropanol verdünnt und sie wurde mit 30%iger Natronlauge auf einen pH-Wert von 8,5 eingestellt.
d) Der Reaktorinhalt wurde unter Rühren auf -10°C abgekühlt und das Kristallisat abgesaugt und mit etwa 1300g kaltem Isopropanol nachgewaschen. Die Ausbeute betrug etwa 88% bezogen auf das eingesetzte Nitrophenol. Das getrocknete Kristallisat ist lagerstabil.

### 2. Stufe: Herstellung von 3,4-dihydro-3-methyl-2H-1,4-benzoxazine (3-MBM)

2624g o-Nitrophenoxyaceton-Kristallisat (~98%ig) aus der 1. Stufe Schritt d) wurden bei Raumtemperatur in einem Gemisch von 11307g Methanol und 1290g Toluol bei 40°C gelöst, um eine etwa 17,6%ige Lösung Nitrophenoxyaceton zu erhalten.

Diese Lösung wurde mit 30%iger wässriger NaOH-Lösung auf einen pH-Wert von 8-9 unter Verwendung einer Schott-Glaselektrode vom Typ H63 eingestellt. Hierfür war nur wenig NaOH notwendig (unter 5 ml). Der pH-Wert der Eduktlösung veränderte sich im Laufe der Zeit und musste vor dem weiteren Einsatz wieder auf einen pH-Wert von 8-9 eingestellt werden. Die Eduktlösung ist in der vorliegenden Form nicht lagerstabil (bei einer Temperatur von 40°C sollte die Lösung nicht länger als wenige Stunden gehalten werden) und mit der Hydrierung sollte zügig begonnen werden

Die erhaltene Lösung wurde kontinuierlich hydriert. Hierzu wurde in einem Autoklaven der Sponge-Metal-Katalysator Amperkat Ni-Mo 3706 in Methanol vorgelegt und unter Wasserstoffdruck und unter Rühren die oNPA-Lösung kontinuierlich zudosiert. Der Reaktor besaß am Deckel eine Fritte, durch die die hydrierte Lösung kontinuierlich durch die frisch einströmende Lösung ausgedrückt wurde.

Reaktionsbedingungen: 2,2 · 10⁷ Pa (220 bar) Wasserstoff, 60°C Reaktionstemperatur bei einer Verweilzeit von etwa 60-90 Minuten. Die Belastung betrug etwa 2g o-Nitrophenoxyaceton pro g eingesetztem Katalysator und Stunde. Der verwendete Katalysator war ein Nickel-Sponge-metal Katalysator der Firma H.C. Starck, Typ Amperkat Ni-Mo 3706 (Austausch des Wassers, unter dem der Katalysator gelagert wird, gegen Methanol). Die Ausbeuten in diesem Schritt lagen bei 95+%, es konnten mindestens 100g Nitrophenoxyaceton pro Gramm rechnerisch trockenem Katalysator umgesetzt werden. Für eine gute Durchmischung wurde durch Rühren gesorgt. Um Ausfällungen der Eduktlösung zu vermeiden, wurde diese auf 40°C temperiert.

Im Laufe der Hydrierung wurden alle zwei Stunden Proben gezogen und per GC-Analytik analysiert. Die niedrigere Konzentration zu Beginn der Reaktion ergab sich durch das Vorlegen von reinem Lösungsmittel zusammen mit dem Katalysator in den Autoklaven, in die dann die eigentliche Reaktionslösung zudosiert wurde. Der rechnerische Wert für einen 100%igen Umsatz lag bei etwa 13,5 % 3MBM in der Lösung.

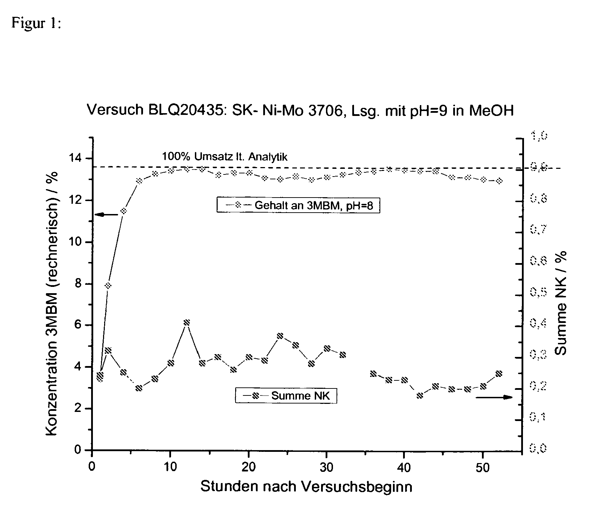

Die Ergebnisse der Hydrierung werden in der folgenden Tabelle dargestellt (siehe auch Figur 1):

| **Stunden nach Versuchsbeginn** | **Gehalt der Lösung an 3MBM / %** | **Nebenkomponenten / % bez. auf 3MBM** | **Menge zudosiertes oNPA / g** |
|---|---|---|---|
| 1 | 3,42 | 7,0 | 32 |
| 2 | 7,9 | 3,7 | 85 |
| 4 | 11,47 | 1,8 | 138 |
| 6 | 12,91 | 1,2 | 249 |
| 8 | 13,26 | 1,4 | 341 |
| 10 | 13,41 | 2,0 | 437 |
| 12 | 13,49 | 2,9 | 538 |
| 14 | 13,5 | 1,7 | 643 |
| 16 | 13,22 | 1,8 | 750 |
| 18 | 13,31 | 1,5 | 855 |
| 20 | 13,33 | 1,8 | 961 |
| 22 | 13,08 | 2,1 | 1070 |
| 24 | 13,04 | 2,8 | 1173 |
| 26 | 13,17 | 2,4 | 1280 |
| 28 | 13,01 | 2,0 | 1385 |
| 30 | 13,15 | 2,4 | 1477 |
| 32 | 13,25 | 2,3 | 1541 |
| 34 | 13,43 | 7,9 | 1619 |
| 36 | 13,43 | 1,8 | 1713 |
| 38 | 13,54 | 1,6 | 1790 |
| 40 | 13,48 | 1,6 | 1874 |
| 42 | 13,46 | 1,3 | 1948 |
| 44 | 13,45 | 1,5 | 2017 |
| 46 | 13,11 | 1,5 | 2115 |
| 48 | 13,14 | 1,5 | 2214 |
| 50 | 13,02 | 1,5 | 2314 |
| 52 | 12,97 | 1,8 | 2409 |

Die aus der Hydrierung erhaltene rote Produktlösung wurde filtriert und das Filtrat analysiert, um den Gehalt an 3-MBM zu bestimmen.

Dann wurde die Lösung in einem Doppelmantelgefäß bei 74°C Sumpftemperatur und etwa 1,8 ·10⁴ Pa (180mbar) Druck vom Lösungsmittel befreit, als Rückstand verblieb eine braune Flüssigkeit, die im wesentlichen aus 3MBM und Wasser bestand. Anschließend wurde in einer Stickstoffatmosphäre weitergearbeitet.

Die dunkelbraune Flüssigkeit wurde, entsprechend dem Analysenergebnis, bei etwa 50°C so mit Toluol verdünnt, dass eine 50%ige Lösung entstand (etwa 3370 g Toluol). Diese Lösung wurde für die Acylierung im folgenden Schritt zur Herstellung von 4-(Dichloracetyl)-3,4-dihydro-3-methyl-2H-1,4-benzoxazin (Benzoxacor) mit einer Ausbeute > 90% eingesetzt..

### Vergleichsbeispiel a. Stufe 2: Herstellung von 3,4-dihydro-3-methyl-2H-1,4-benzoxazine (3-MBM) ohne vorhergehende Kristallisation und ohne pH-Wert Einstellung und bei niedrigerem Druck von 1,5·10⁷ Pa (150 bar) bei höhere Temperatur von 80°C

Anstelle der in Schritt 2 verwendeten kristallisierten Ware aus Schritt 1d) wird in diesem Vergleichsbeispiel die organische Lösung aus Schritt 1b) eingesetzt, die mit 8500g Methanol verdünnt wurde, um die Löslichkeit der o-Nitrophenoxyacetons bei 40°C zu gewährleisten. So wurde eine Lösung erhalten, die etwa 20% an o-Nitrophenoxyacetons enthielt.

Die erhaltene Lösung wurde kontinuierlich hydriert. Hierzu wurde in einem Autoklaven der Sponge-Metal-Katalysatoren Amperkat Ni-Mo 3706 in Methanol vorgelegt und unter Wasserstoffdruck und unter Rühren die oNPA-Lösung kontinuierlich zudosiert. Der Reaktor besaß am Deckel eine Fritte, durch die die hydrierte Lösung kontinuierlich durch die frisch einströmende Lösung ausgedrückt wurde.

Reaktionsbedingungen: **1,5 ·10⁷ Pa** (150 bar) Wasserstoff, 80°C Reaktionstemperatur bei einer Verweilzeit von etwa 60-90 Minuten. Die Belastung betrug etwa 2g o-Nitrophenoxyaceton pro g eingesetztem Katalysator und Stunde. Der verwendete Katalysator war ein Sponge-metal Katalysator der Firma H.C. Starck, Typ Amperkat Ni-Mo 3706 (Austausch des Wassers, unter dem der Katalysator gelagert wird, gegen Methanol). Die Ausbeute lag bei 90%, es konnten 42g Nitrophenoxyaceton pro Gramm rechnerisch trockenem Katalysator umgesetzt werden. Danach musste die Hydrierung aufgrund der massiven Bildung von Nebenkomponenten (vor allem des 3MBM-Dimeren) abgebrochen werden. Für eine gute Durchmischung wurde durch Rühren gesorgt. Um Ausfällungen der Eduktlösung zu vermeiden, wurde diese auf 40°C temperiert.

Im Laufe der Hydrierung wurden alle zwei Stunden Proben gezogen und per GC-Analytik analysiert. Die niedrigere Konzentration zu Beginn der Reaktion ergab sich durch das Vorlegen von reinem Lösungsmittel zusammen mit dem Katalysator in den Autoklaven, in die dann die eigentliche Reaktionslösung zudosiert wurde. Der rechnerische Wert für einen 100%igen Umsatz lag bei etwa 15,2 % 3MBM in der Lösung.

Die Ergebnisse der Hydrierung werden in der folgenden Tabelle dargestellt:

| **Stunden nach Versuchsbeginn** | **Gehalt der Lösung an 3MBM / %** | **Summe Hauptnebenkomponenten / % bez. auf 3MBM** | **Menge zudosiertes oNPA-Lösung / g** |
|---|---|---|---|
| 1 | 1,97 | 20,8 | 194 |
| 2 | 7,57 | 2,5 | 375 |
| 4 | 12,6 | 4,5 | 599 |
| 6 | 14,9 | 6,6 | 823 |
| 8 | 16,43 | 8,5 | 1799 |
| 10 | 16,46 | 5,5 | 2679 |
| 12 | 16,63 | 7,6 | 3532 |
| 14 | 16,52 | 8,0 | 3931 |
| 16 | 16,36 | 9,1 | 4178 |
| 18 | 16,13 | 9,7 | 4502 |
| 20 | 15,7 | 10,6 | 4806 |
| 22 | 14,89 | 9,0 | 5922 |
| 24 | 15 | 10,1 | 7287 |
| 26 | 14,54 | 10,5 | 8937 |
| 28 | 14,31 | 9,2 | 9227 |

Die aus der Hydrierung erhaltene rote Produktlösung wurde filtriert und das Filtrat analysiert, um den Gehalt an 3-MBM zu bestimmen.
Die in der erhaltenen Lösung enthaltenen Nebenkomponenten sind so hoch, dass die Darstellung von ausreichend reinem Benoxacor unter diesen Produktionsbedingungen nicht möglich ist.

### Vergleichsbeispiel b, Stufe 2: Herstellung von 3,4-dihydro-3-methyl-2H-1,4-benzoxazine (3-MBM) ohne vorhergehende Kristallisation und ohne pH-Wert Einstellung und bei Druck von 2,2 ·10⁷ Pa (220bar) bei 60°C

Anstelle der in Schritt 2 verwendeten kristallisierten Ware aus Schritt 1d) wurde in diesem Vergleichsbeispiel die organische Lösung aus Schritt 1b) eingesetzt, die mit 15343g Methanol verdünnt wurde, um die Löslichkeit der o-Nitrophenoxyacetons bei 40°C zu gewährleisten.
So wurde eine Lösung erhalten, die etwa 16,4% o-Nitrophenoxyacetons enthielt.

Die erhaltene Lösung wurde kontinuierlich hydriert. Hierzu wurde in einem Autoklaven der Sponge-Metal-Katalysatoren Amperkat Ni-Mo 3706 in Methanol vorgelegt und unter Wasserstoffdruck und unter Rühren die oNPA-Lösung kontinuierlich zudosiert. Der Reaktor besaß am Deckel eine Fritte, durch die die hydrierte Lösung kontinuierlich durch die frisch einströmende Lösung ausgedrückt wurde.

Reaktionsbedingungen: **2,2 ·10⁷ Pa** (220 bar) Wasserstoff, 60°C Reaktionstemperatur bei einer Verweilzeit von etwa 60-90 Minuten. Die Belastung betrug etwa 2g o-Nitrophenoxyaceton pro g eingesetztem Katalysator und Stunde. Der verwendete Katalysator war ein Nickel-Sponge-metal Katalysator der Firma H.C. Starck, Typ Amperkat Ni-Mo 3706 (Austausch des Wassers, unter dem der Katalysator gelagert wird, gegen Methanol). Die Ausbeute zu 3MBM lag bei etwa 74%, es konnten über 40g Nitrophenoxyaceton pro Gramm rechnerisch trockenem Katalysator umgesetzt werden. Danach musste die Hydrierung aufgrund der massiven Bildung von Nebenkomponenten (vor allem einer Unbekannten Verbindung mit Masse 211 amu) abgebrochen werden. Für eine gute Durchmischung wurde durch Rühren gesorgt. Um Ausfällungen der Eduktlösung zu vermeiden, wurde diese auf 40°C temperiert.

Im Laufe der Hydrierung wurden alle zwei Stunden Proben gezogen und per GC-Analytik analysiert. Die niedrigere Konzentration zu Beginn der Reaktion ergab sich durch das Vorlegen von reinem Lösungsmittel zusammen mit dem Katalysator in den Autoklaven, in die dann die eigentliche Reaktionslösung zudosiert wurde. Der rechnerische Wert für einen 100%igen Umsatz lag bei etwa 12,5 % 3MBM in der Lösung.

Die Ergebnisse der Hydrierung werden in der folgenden Tabelle dargestellt:

| **Stunden nach Versuchsbeginn** | **Gehalt der Lösung an 3MBM / %** | **Summe Hauptnebenkomponenten / % bez. auf 3MBM** |
|---|---|---|
| 1 | 0,59 | 62,7 |
| 2 | 3,43 | 93,0 |
| 4 | 6,93 | 68,7 |
| 6 | 8,8 | 58,4 |
| 8 | 9,35 | 52,7 |
| 10 | 9,43 | 51,0 |
| 12 | 9,38 | 52,6 |
| 14 | 9,23 | 53,3 |
| 16 | 9,36 | 55,1 |
| 18 | 9,31 | 54,1 |
| 20 | 9,31 | 54,9 |
| 21 | 9,26 | 55,1 |

Die aus der Hydrierung erhaltene rote Produktlösung wurde filtriert und das Filtrat analysiert, um den Gehalt an 3-MBM zu bestimmen.

Die in der erhaltenen Lösung enthaltenen Nebenkomponenten sind so hoch, dass die Darstellung von ausreichend reinem Benoxacor unter diesen Produktionsbedingungen nicht möglich ist.

### 3. Stufe: 4-(dichloracetyl)-3,4-dihydro-3-methyl-2H-1,4-benzoxazine (Benoxacor)

3624 g einer 50%igen 3MBM-Lösung aus Stufe 2 wurde mit 2048g Wasser und 53,2g H₃PO₄ 75%ig versetzt

Unter Rühren wurde nun simultan eine Lösung von 2519g 96%iges DCAC in 2120g Toluol und eine 30%ige NaOH Lösung bei 40-70°C in 90 min so zudosiert, dass der pH-Wert zwischen 2-3 gehalten wurde.

Die Emulsion wurde auf 80°C erhitzt und 1 h bei 80°C nachgerührt.

Dann wurde die Emulsion mit 30%iger NaOH auf pH 7 gestellt und unter Zusatz von Cellulose heiß filtriert.

Die untere wässrige Phase wurde bei 80°C abgetrennt und die org. Phase bis 95°C Manteltemperatur und **2,5· 10⁴ Pa** (250 mbar) vom Lösungsmittel befreit.

Der Rückstand wurde bei 80°C mit 8100g Isopropanol versetzt und darin gelöst.

Die Lösung wurde dann schrittweise mit insgesamt 12000 g Wasser versetzt, bis sie sich eintrübte. Nach Abkühlung auf Raumtemperatur wurde das Kristallisat abgesaugt und mit 15000 kg Isopropanol/Wasser-Gemisch (10-50Gew-% Isopropanol, bevorzugt 10 Gew.-%) gewaschen.

Abschließend wurde noch mit 9000 g Wasser nachgewaschen.

Die Ausbeute betrug 91% bezogen auf das eingesetzte 3MBM. Das so erhaltene Benoxacor zeigte analytisch einen Gehalt von > 99,8 %.

## Patentansprüche

1. Verfahren zur Herstellung einer Acylamid-Verbindung der allgemeinen Formel (I) 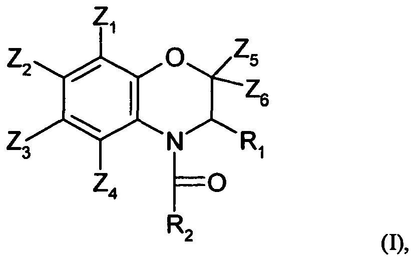 in der
R₁ Wasserstoff oder ein C₁-C₈-Alkylrest, R₂ eine Dichlormethyl- oder Trichlorethylgruppe und Z₁ bis Z₆ jeweils unabhängig voneinander Wasserstoff oder ein C₁-C₈-Alkylrest sind,
**dadurch gekennzeichnet, dass** man
a) eine o-Nitrophenoxycarbonylverbindung der allgemeinen Formel (II) 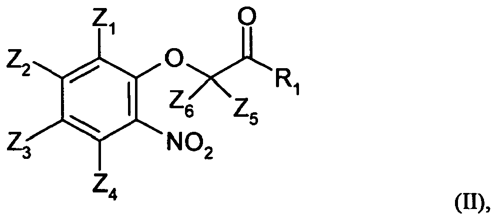 in der
R₁ und Z₁ bis Z₆ die für Formel (I) angegebene Bedeutung haben,
aus einem organischen Lösungsmittel auskristallisieren lässt und abtrennt,
b) die o-Nitrophenoxycarboxylverbindung in einem Gemisch aus einem C₁-C₃-Alkohol und einem aromatischen Lösungsmittel löst,
c) den pH-Wert in dieser Lösung auf einen Wert zwischen 8 und 9 einstellt,
d) die in dieser Lösung enthaltene o-Nitrophenoxyverbindung der Formel (II) in Gegenwart eines Sponge-metal-Katalysators mit Wasserstoff-Gas unter Ringschluss zur Benzoxazin-Verbindung der Formel (III) hydriert 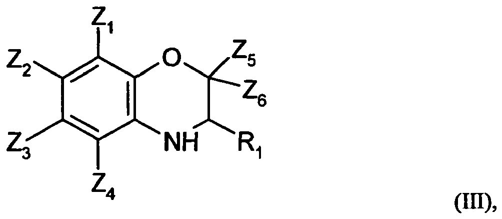 in der R₁ und Z₁ bis Z₆ die für Verbindungen der Formel(I) angegeben Bedeutung haben, und dann
e) die Verbindung der Formel (III) mit einem Acylhalogenid der Formel (IV) 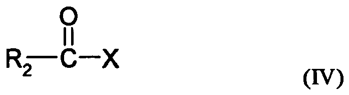
in der R₂ die für Verbindungen der Formel (I) angegebene Bedeutung hat und X für ein Halogenatom steht, zu Verbindungen der Formel (I) umsetzt.

2. Verfahren nach Anspruch 1, **dadurch gekennzeichnet, dass** der Katalysator ein mit Molybdän und/oder Aluminium und/oder Eisen dotierter Sponge-metal-Katalysator auf der Basis von Nickel ist.

3. Verfahren nach Anspruch 1 oder 2, **dadurch gekennzeichnet, dass** man in Schritt a) die o-Nitrophenoxycarbonylverbindung der Formel (II) aus einem Lösungsmittel aus der Gruppe Ethanol, Propanol, Isopropanol auskristallisieren lässt.

4. Verfahren nach einem der Ansprüche 1 bis 3, **dadurch gekennzeichnet, dass** in Schritt d) der Druck des Wasserstoff-Gases im Bereich zwischen 150 und 240 bar (1,5·10⁷ und 2,4·10⁷PA) liegt.

5. Verfahren nach einem der Ansprüche 1 bis 4, **dadurch gekennzeichnet, dass** in Schritt d) die Reaktionstemperatur während der Hydrierung im Bereich zwischen 30 und 90°C liegt.

6. Verfahren nach einem der Ansprüche 1 bis 5, **dadurch gekennzeichnet, dass** die Hydrierung in einem Lösungsmittelgemisch aus Methanol und Toluol durchgeführt wird.

7. Verfahren nach einem der Ansprüche 1 bis 6, **dadurch gekennzeichnet, dass** die Acylamid-Verbindung der Formel (I) 4-(Dichloracetyl)- 3,4-Dihydro-3-methyl-2H-1,4-benzoxazin ist (R₁ = Methyl , R2 = Dichlormethyl und Z₁ bis Z₆ jeweils Wasserstoff).

## Claims

1. Process for preparing an acyl amide compound of the general formula (I) 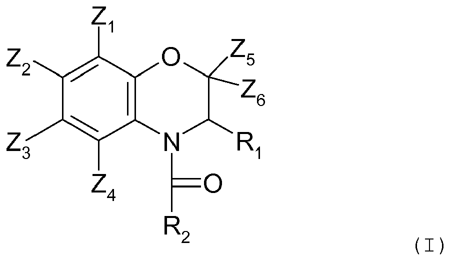 in which
R₁ is hydrogen or a C₁-C₈-alkyl radical, R₂ is a dichloromethyl or trichloroethyl group, and Z₁ to Z₆ are each independently hydrogen or a C₁-C₈-alkyl radical,
**characterized in that**
a) an o-nitrophenoxy carbonyl compound of the general formula (II) 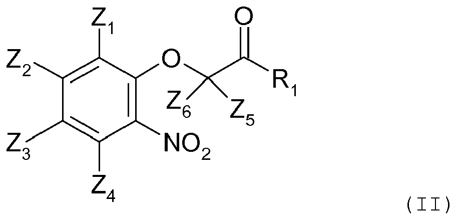 in which
R₁ and Z₁ to Z₆ are each as defined for formula (I)
is allowed to crystallize out of an organic solvent and removed,
b) the o-nitrophenoxy carbonyl compound is dissolved in a mixture of C₁-C₃-alcohol and an aromatic solvent,
c) the pH in this solution is set to a value between 8 and 9,
d) the o-nitrophenoxy compound of the formula (II) present in this solution is hydrogenated in the presence of a sponge metal catalyst with hydrogen gas with ring closure to give the benzoxazine compound of the formula (III) 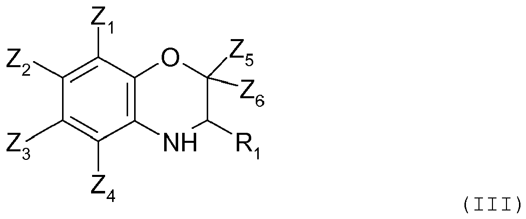 in which R₁ and Z₁ to Z₆ are each as defined for compounds of the formula (I), and then
e) the compound of the formula (III) is reacted with an acyl halide of the formula (IV) 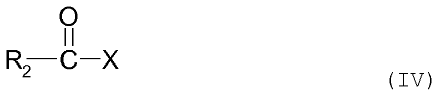
in which R₂ is as defined for compounds of the formula (I) and X is a halogen atom to give compounds of the formula (I).

2. Process according to Claim 1, **characterized in that** the catalyst is a molybdenum- and/or aluminium- and/or iron-doped sponge metal catalyst based on nickel.

3. Process according to Claim 1 or 2, **characterized in that**, in step a), the o-nitrophenoxy carbonyl compound of the formula (II) is allowed to crystallize out of a solvent from the group of ethanol, propanol, isopropanol.

4. Process according to any of Claims 1 to 3, **characterized in that**, in step d), the pressure of the hydrogen gas is in the range between 150 and 240 bar (1.5·10⁷ and 2.4 ·10⁷ Pa).

5. Process according to any of Claims 1 to 4, **characterized in that**, in step d), the reaction temperature during the hydrogenation is between 30 and 90°C.

6. Process according to any of Claims 1 to 5, **characterized in that** the hydrogenation is performed in a solvent mixture of methanol and toluene.

7. Process according to any of Claims 1 to 6, **characterized in that** the acyl amide compound of the formula (I) is 4-(dichloroacetyl)-3,4-dihydro-3-methyl-2H-1,4-benzoxazine (R₁ methyl, R₂ = dichloromethyl and Z₁ to Z₆ each hydrogen).

## Revendications

1. Procédé pour la préparation d'un composé de type acylamide de formule générale (I) 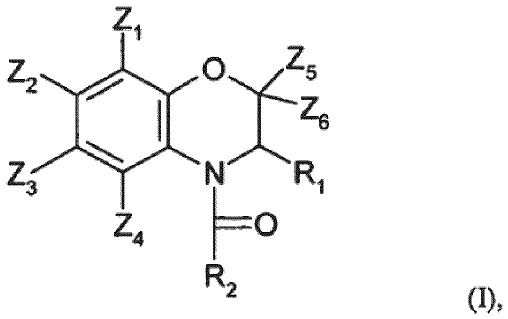 dans laquelle
R₁ est un atome d'hydrogène ou un radical alkyle en C₁-C₈, R² est un groupe dichlorométhyle ou trichloréthyle et Z₁ à Z₆ représentent chacun indépendamment l'un de l'autre un atome d'hydrogène ou un groupe alkyle en C₁-C₈,
**caractérisé en ce que**
a) on fait cristalliser dans un solvant organique un composé o-nitrophénoxycarbonyle de formule générale (II) 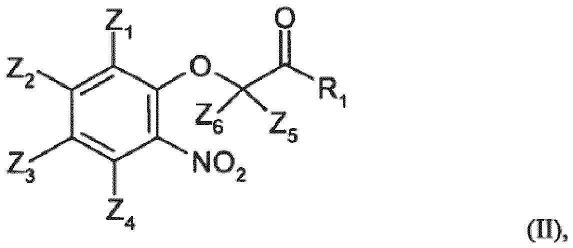 dans laquelle
R₁ et Z₁ à Z₆ ont les significations indiquées pour la formule (I),
et on le sépare,
b) on dissout le composé o-nitrophénoxycarbonyle dans un mélange d'un alcool en C₁-C₃ et d'un solvant aromatique,
c) on ajuste le pH dans cette solution à une valeur comprise entre 8 et 9,
d) on soumet le composé o-nitrophénoxy de formule (II), contenu dans cette solution, à une hydrogénation avec du gaz hydrogène en présence d'un catalyseur métallique spongieux, avec cyclisation en le composé de type benzoxazine de formule (III) 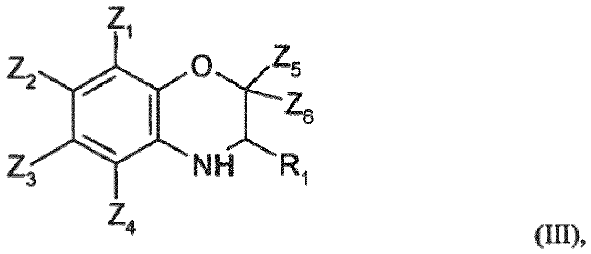 dans laquelle R₁ et Z₁ à Z₆ ont les significations indiquées pour les composés de formule (I),
et ensuite
e) on fait réagir le composé de formule (III) avec un halogénure d'acyle de formule (IV) 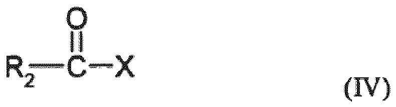
dans laquelle R₂ a la signification indiquée pour les composés de formule (I) et X représente un atome d'halogène, pour aboutir à des composés de formule (I).

2. Procédé selon la revendication 1, **caractérisé en ce que** le catalyseur est un catalyseur métallique spongieux à base de nickel, dopé avec du molybdène et/ou de l'aluminium et/ou du fer.

3. Procédé selon la revendication 1 ou 2, **caractérisé en ce que** dans l'étape a) on fait cristalliser le composé o-nitrophénoxycarbonyle de formule (II) dans un solvant choisi dans le groupe constitué par l'éthanol, le propanol, l'isopropanol.

4. Procédé selon l'une quelconque des revendications 1 à 3, **caractérisé en ce que** dans l'étape d) la pression du gaz hydrogène se situe dans la plage comprise entre 150 et 240 bars (1,5.10⁷ et 2,4.10⁷ Pa).

5. Procédé selon l'une quelconque des revendications 1 à 4, **caractérisé en ce que** dans l'étape d) la température de réaction pendant l'hydrogénation se situe dans la plage comprise entre 30 et 90 °C.

6. Procédé selon l'une quelconque des revendications 1 à 5, **caractérisé en ce qu'**on effectue l'hydrogénation dans un mélange de solvants constitué de méthanol et toluène.

7. Procédé selon l'une quelconque des revendications 1 à 6, **caractérisé en ce que** le composé de type acylamide de formule (I) est la 4-(dichlor-acétyl)-3,4-dihydro-3-méthyl-2H-1,4-benzoxazine (R₁ = méthyle, R₂ = dichlorométhyle et Z₁ à Z₆ représentent chacun un atome d'hydrogène).
